(19) **Europäisches Patentamt**
**European Patent Office**
**Office européen des brevets**

(11) **EP 2 822 383 B1**

(12) **EUROPEAN PATENT SPECIFICATION**

(45) Date of publication and mention
of the grant of the patent:
**02.05.2018 Bulletin 2018/18**

(21) Application number: **13720543.1**

(22) Date of filing: **07.03.2013**

(51) Int Cl.:
*A01N 37/02* (2006.01)     *A61K 31/20* (2006.01)
*C11C 1/04* (2006.01)      *A01P 1/00* (2006.01)
*C11B 3/02* (2006.01)      *A23K 20/158* (2016.01)
*A23K 20/195* (2016.01)    *A23K 50/10* (2016.01)
*A23K 50/80* (2016.01)     *A23K 50/60* (2016.01)

(86) International application number:
**PCT/IB2013/000629**

(87) International publication number:
**WO 2013/132334 (12.09.2013 Gazette 2013/37)**

(54) **ANTIMICROBIAL COMPOSTION CONTAINING LAURIC ACID AND METHOD FOR THEIR PRODUCTION**

ANTIMIKROBIELLE ZUSAMMENSETZUNG ENTHALTENDEN LAURINSÄURE UND VERFAHREN ZUR DESSEN HERSTELLUNG

COMPOSITION ANTIMICROBIENNE CONTENANT D'ACIDE LAURIQUE ET SA PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2012 EP 12001554**

(43) Date of publication of application:
**14.01.2015 Bulletin 2015/03**

(73) Proprietor: **Cargill Inc.**
**Wayzata, MN 55391 (US)**

(72) Inventors:
• **HOLLANDER, Frank**
**NL-4051 BL Ochten (NL)**
• **KRUIDENBERG, Marcus, Bernardus**
**NL-3233 SL Oostvoome (NL)**
• **LOBEE, Henricus, Wilhelmus, Jozef**
**NL-5305 CD Zuilichem (NL)**
• **VAN DER HOEVEN-HANGOOR, Evelien**
**Istanbul (TR)**

(74) Representative: **Elseviers, Myriam**
**Cargill R&D Centre Europe BVBA**
**Bedrijvenlaan 9**
**2800 Mechelen (BE)**

(56) References cited:
**WO-A2-2011/012440     US-A- 4 223 040**
**US-A- 5 142 071        US-A1- 2012 041 065**

• **Anonymous: "Exporter of distilled coconut fatty acid", ECPlaza , 4 October 2008 (2008-10-04), XP002681815, Retrieved from the Internet: URL:http://www.ecplaza.net/trade-leads-sel ler/exporter-of-distilled-coconut-fatty--5 352705.html [retrieved on 2012-08-13]**
• **Anonymous: "Edenor HK 8-18", Oleochemicals , 21 May 2007 (2007-05-21), pages 1-2, XP055035357, Retrieved from the Internet: URL:http://cognis.plansbiz.net/imgs/upload s/1file__08201003728.pdf [retrieved on 2012-08-13]**
• **WIM DE GREYT: "Developments in Edible Oil Refining for the Production of High Quality Food Oils", 101ST AOCS ANNUAL MEETING, 19 May 2010 (2010-05-19), XP055035474, Phoenix, Arizona**
• **J. J. Kabara ET AL: "Fatty Acids and Derivatives as Antimicrobial Agents", Antimicrobial agents and chemotherapy, vol. 2, no. 1, 1 July 1972 (1972-07-01), pages 23-28, XP055082709, ISSN: 0066-4804, DOI: 10.1128/AAC.2.1.23**

EP 2 822 383 B1

**(Cont. next page)**

• Daniela I Batovska ET AL: "Antibacterial study of the medium chain fatty acids and their 1-monoglycerides: individual effects and synergistic relationships.", Polish Journal of Microbiology, vol. 58, no. 1, 1 January 2009 (2009-01-01), pages 43-47, XP55035299,

## Description

### TECHNICAL FIELD

The present invention relates to antimicrobial C6-C12 fatty acids compositions as defined in claim 1 for improving feed efficiency. In particular, it relates to compositions which can be used to prevent the growth of microbial agents in animals and improve feed efficiency.

### BACKGROUND OF THE INVENTION

Medium-chain fatty acids (MCFAs), i.e. fatty acids with a carbon chain length from 6-12 carbon atoms, are considered to be a unique category of fat substances. Unlike long-chain fatty acids, MCFAs can be absorbed directly into the bloodstream without re-esterification or inclusion in 15 chylomicrons. As such, MCFAs can be transported rapidly to organs requiring energy. What's more, MCFAs are preferentially oxidized in the mitochondria, making them an excellent source of fast energy.

Certain MCFAs have also been found to have a beneficial antimicrobial effect. This is 20 considered a key attribute in the field of animal husbandry where controlling levels of microorganisms in the animals' digestive tract is a priority. Animals may be subject to exposure to bacteria, yeast and fungi through the rearing environment and feed products. These microorganisms can cause significant disruption to the animals' digestive system and an imbalance in the microbial ecosystem of their gastrointestinal tract. This can result in less efficient digestion 25 and nutrient absorption which, in turn, will affect growth rates. It could also lead, in some cases, to disease and, potentially, to the loss of the animal. In any event, it is clear that being able to control microbial populations has a significant effect on profitability. This used to be achieved through the application of low-dose antibiotics. However, the addition of such growth promoters to feed products was banned, in the EU, in 2006. Interest in natural alternatives - such as 30 MCFAs - has therefore increased.

Laurie oils, such as coconut oil and palm kernel oil, are known to be rich in MCFAs. Unfortunately, triglycerides containing these fatty acids do not have the observed antimicrobial activity themselves. The MCFAs have to be separated from their glycerol backbone and used in 35 their free fatty acid (FFA) form. This is achieved, in the industry, by splitting crude lauric oils and then distilling the oil to obtain a FFA fraction and a glycerine fraction.

There are at least four known methods of fat splitting: the Twitchell process (although somewhat archaic now), the batch autoclave process, the continuous countercurrent process, and the enzymatic process (using lipase enzymes). The fatty acids produced by these processes are 5 then purified and separated into fractions by distillation and fractionation.

Because of the extreme sensitivity of fatty acids to heat, oxidation and corrosion effects, distillation must be performed under highly controlled conditions - i.e. under high vacuum, at lower temperatures and with the shortest possible residence time. Nonetheless, fatty acid 10 distillates tend to develop a strong taste and smell (caused by secondary oxidation products such as ketones and aldehydes). They can also have a strong degree of coloration due to the presence of pigments such as carotenoids. This process may also result in concentrations of contaminants (such as dioxins and poly aromatic hydrocarbons (PAHs)) which are very difficult to remove.

Nonetheless, these distillates have been considered generally acceptable for animal feed. There is, however, increasing concern over contaminant levels. What's more, their strong odor and taste has always made them unsuitable for use in certain animal feeds: aquatic feed (e.g. for fish and shrimp), feed for young animals (e.g. veal calves and piglets), and domestic animal food all require the use of less strong-tasting and -smelling oils-

"Distilled coconut fatty acid" specification discloses distilled coconut fatty acid having Lovibond red colour of max. 1.

"Edenor HK 8-18" specification discloses hydrogenated and hydrolysed coconut oil with Lovibond colour red 0.2.

US 5142071 A discloses the selective esterification of long chain fatty acid monoglycerides with medium chain fatty acids..

WO 2011/012440 A2 discloses crystallization process for the production of bio-naphat from complex mixtures of natural occurring fats and oils. The complex mixture is subjected to a refining treatment for removing the major part of the non-triglyceride and non-fatty acid components.

US 2012/041065 A1 relates to lauric acid distillate for animal feed.

US 4223040 A discloses the use of lauric acid for treatment of mycobacterial diseases.

"Developments in Edible Oil Refining for the Production of High Quality Food Oils", 101st AOCS ANNUAL MEETING discloses process options for edible oil refining.

Antimicrobial agents and chemotherapy, vol. 2, no. 1, p. 23-28 and Polish Journal of Microbiology, vol. 58, no. 1, p. 43-47 disclose antibacterial activities of medium chain fatty acids.

There is therefore a clear need in the art for a new "natural" antimicrobial agent with better or less taste, smell and color and with a reduced contaminant content - and for a process for producing such products. The present invention addresses this need.

## STATEMENTS OF THE INVENTION

According to a first aspect of the present invention, there is provided an antimicrobial composition obtainable by a method comprising the step of splitting a refined lauric oil characterized in that the refined oil is an oil that has been brought into contact with activated carbon, said composition comprising 90% or more, by weight, free fatty acids, FFAs, and characterized in that it comprises, by weight: 0-5% C6 fatty acids; 1-15% C8 fatty acids; 1-15% C10 fatty acids; and 35-70% C12 fatty acids; and having a total PAH (polycyclic aromatic hydrocarbons) level of 20 TEQ B[a]P (toxicity equivalence of sum of benzo[a]pyrene, benzo[a]anthracene, benzo[b]fluorthene and chrysene), or less.

According to another aspect of the present invention, there is provided an antimicrobial composition as defined above for use in preventing the growth of microbial agents and/or for improving gastro-intestinal health in animals,; and/or for use in increasing feed efficiency, and/or for use in enhancing growth and/or reducing mortality in animals.

According to a yet further aspect of the present invention, there is provided a feed, food or beverage composition comprising the antimicrobial composition defined above, preferably in an amount of 0.05-10% by weight, based on total weight of the feed, food or beverage composition.

## DESCRIPTION OF INVENTION

The present invention provides a method of producing antimicrobial compositions. The term "antimicrobial" as used herein refers to substances that are capable of killing or inhibiting the growth of microorganisms such as bacteria and fungi (including yeasts). In particular, it may refer to substances that are capable of killing or inhibiting the growth of fungi such as Aspergillus, Candida, Cephalosporum, Fusarium, and Penicillium; yeasts such as Saccharomyces; Gram-negative bacteria such as Escherichia coli, Salmonella, and Shigella; and Gram-positive bacteria such as Listeria.

The composition of the present invention is obtained by the process which includes the step of splitting an activated carbon treated lauric oil to obtain a composition comprising 90% or more, by weight, free fatty acids. Laurie oils will generally be understood to be oils rich in MCFAs, as defined below (i.e. comprising at least 40%, preferably at least 50%, more preferably at least 60% MCFAs by weight). Examples of such oils include coconut oil, palm kernel oil, babassu oil, cohune oil, tacum oil and cuphea oil, together with any fractions (especially olein fractions) or derivatives (obtained, e.g. by full or partial hydrogenation) thereof. For the purposes of the present invention, the lauric oil will preferably a coconut oil or coconut olein fraction, a palm kernel oil or palm kernel olein fraction, or mixtures of two or more thereof.

Activated carbon treatment may be performed with powdered, granular, extruded or bead activated

carbon - or with any other known form of activated carbon. The treatment may be performed in a continuous or batch process. Preferably, it will be performed in a column with granular activated carbon. The oil will preferably be brought into contact with the activated carbon at a temperature of 50-120°C, preferably of 50-90°C, more preferably of 50-75°C. For example, the oil may be brought into contact with the activated carbon at a temperature of about 5 60°C. The activated carbon may be used, for instance, in an amount of 10g per MT of oil to 50 kg per MT of oil, preferably in an amount of 100g per MT of oil to 25 kg per MT of oil, more preferably in an amount of 500g per MT of oil to 10 kg per MT of oil, more preferably in an amount of 750g per MT of oil to 5 kg per MT of oil. According to one aspect of the invention, it will be used in an amount of 1-2 kg per MT of oil. Other parameters and variations thereof will be 10 apparent to a person skilled in the art.

The lauric oils used in **splitting process are** pre-treated with activated carbon or the process may itself include a step of bringing the oils into contact with activated carbon to obtain an activated carbon treated lauric oil.

Activated carbon treatment may be used on its own or, alternatively, it may be used in combination with one or more refining steps. Indeed, although certain crude lauric oils (such as some sun-dried coconut oil from the Fuji Islands) have very low contaminant levels and can thus be used as such in the method **described above,** most crude oils (i.e. oils as extracted 20 from their original source) will preferably be refined. These oils have high levels of contaminants - such as phosphatides, soaps and pigments - which may cause an undesirable colour, odor or taste. These oils may be refined before use in the process as **described above** or the process may itself include one or more refining steps.

Refining typically consists of three major processes: degumming, bleaching and deodorization, all of which are well known processes to a person skilled in the art. According to one aspect of the present invention, the lauric oils used in the present invention will have been subjected to one or more of (and preferably all of) degumming, bleaching and deodorization. Alternatively, the process of **preparing the antimicrobial composition of the** present invention may itself include one or more of (and preferably all of) these 30 steps. Each of the steps may be performed separately or, alternatively, treatment with activated carbon may be performed simultaneously with the bleaching step (e.g. by combining the activated carbon and a bleaching earth together in a single column).Other refining steps may also be used as will be apparent to a skilled person.

For ease of reference, an oil which has been brought into contact with activated carbon, whether or not it has been subjected to one or more additional refining steps, will simply be referred to herein as an "activated carbon treated oil".

The activated carbon treated lauric oil is split such that a composition comprising 90% or more, by weight, free fatty acids is obtained. Unexpectedly, it has been observed that the treatment with activated carbon must be performed prior to the splitting step. Splitting, as described above, is a process for separating fatty acids from their glycerol backbone. A number of splitting techniques are known in the art and do not all need to 10 be described in great detail here. For instance, the splitting technique used in the method of the present invention may encompass chemical hydrolysis and/or enzymatic splitting. By way of illustration only, chemical hydrolysis may be performed in a batch process (e.g. in a batch autoclave process) or in continuous process (also known as the Colgate-Emery process). Enzymatic splitting will preferably be performed with lipase enzymes such as fungal lipase 15 enzymes, e.g. from the Candida, Aspergillus or Rhizopus genera. Specific examples of suitable lipase enzymes are those from Candida Rugosa, Aspergillus niger or Rhizopus arrhizus.

Preferably, splitting will be achieved through chemical hydrolysis under high pressure and at high temperatures. According to one possible embodiment, the split oil may be further subjected 20 to one or more additional steps including, but not limited to, distillation, fractionation and/or purification. Advantageously, however, even without such additional steps, the compositions obtained by the process of the present invention will have a FFA content of at least 90% by weight, as determined according to AOCS method Ca 5a-40 (using the average molecular weight of lauric acid). What's more, they will preferably have an appropriate profile for use as a 25 natural antimicrobial agent in the manufacture of feed, food and beverage compositions.

As mentioned above FFA content is measured using the standard method AOCS Ca 5a-40 (using average molecular weight of lauric acid). Preferably, the compositions recovered from the process of the present invention will comprise 95% or more, more preferably 97% or more, more preferably 99% or more FFA by weight. The FFAs will have a high MCFA content. Preferably, 35 they will comprise more than 50% MCFAs. More preferably, they will comprise 60% or more, 70% or more, or 80% or more MCFAs.

The term "MCFA" as used herein will be understood, in the context of the present invention, as referring to fatty acids with a carbon chain length from 6-12 carbon atoms (i.e. caproic acid (C6), caprylic acid (C8), capric acid (C10) and lauric acid (C12)), as well as to salts, emulsions and the like, together with mixtures thereof. The composition of the present invention comprises by weight:

- 0-5%, preferably 0-1 %, C6 fatty acids;
- 1-15% , preferably 2-10%, C8 fatty acids;
- 1-15%, preferably 1-8%, C10 fatty acids; and
- 35-70%, preferably 40-55%, C12 fatty acids.

It has less color, a better taste and/or smell, and/or fewer contaminants (that is to say a lower concentration of contaminants) than similar antimicrobial compositions obtained by distillation alone. The compositions of the present invention will preferably have:

- a Lovibond red colour of 5 or less; and/or
- an acceptable taste and smell as determined by Method A; in addition to
- a total PAH content of 20 TEQ B[a]P or less; and preferably
- a total dioxin content no greater than 1.5 TEQ.

Ideally, they will have a Lovibond red colour of 5 or less; and an acceptable taste and smell as determined by Method A; and a total PAH level of 20 TEQ B[a]P or less; and a total dioxin content no greater than 1.5 TEQ.

Lovibond red colour is measured on the Lovibond colour scale in accordance with AOCS method Cc13e. Red color is graded on a scale of 0.1 to 20. Preferably, compositions obtained according to the method of the present invention will have a Lovibond Red colour of 5 or less, more preferably of 2 or less, more preferably of 1 or less, for example of 0.5 or less. Ideally, the compositions will be white (when in their solid state).

The antimicrobial composition of the present invention will also preferably be devoid of any burnt taste or smell (which is often associated with e.g. coconut oil due to the processes used to dry the source materials). Taste and odor are assessed according to Method A: AOCS Method Cg

2-83. According to this method, oils are given a score from 1 to 10, wherein 1 indicates an oil with a very strong taste and/or odor, and 10 indicates a tasteless, odorless oil. Compositions will be considered as having an acceptable taste and odor according to the present invention if, when measured according to Method A, they have a score of 7 or more. Preferably, the 5 compositions of the invention will be characterized by a score of 8 or more.

**The** compositions of the present invention will also have very low levels of contaminants. **They** will preferably have reduced levels of polycyclic aromatic hydrocarbons (PAHs) and of dioxins and dioxin-like compounds (DLCs) compared to 10 corresponding oils obtained by distillation and fractionation. For ease of reference, dioxins and DLCs will commonly be referred to herein simply as dioxins.

As toxic compounds all have a different toxic effects, toxicity is typically measured in terms of toxicity equivalence (TEQ). TEQ is calculated as the product of the concentration of an individual 15 toxic compound in a sample (in pg/kg) and its corresponding toxicity equivalency factor (TEF), i.e. as follows:

TEQ of compound A = concentration of A x TEF of A

The TEF of a compound is expressed as a number, between 0 and 1, which represents the compound's relative toxicity compared to that of the most toxic known compound in that category of compounds. Thus, for instance, the TEFs for PAHs are calculated relative to the toxicity of benzo[a]pyrene (B[a]P) - and the PAH content of a composition will therefore be expressed in terms of TEQ B[a]P. The TEFs of dioxins are calculated relative to the toxicity of 25 2,3,7,8-tetrachlorodibenzodioxin (TCDD) - with the dioxin content of a composition conventionally being expressed simply in terms of "TEQ".

The toxicity of a composition - i.e. its total toxicity equivalence - will depend, to a certain extent, on the number of PAHs or dioxins being measured. Regulatory guidelines and food and feed law 30 in different countries will often dictate the number of compounds that must be taken into account when assessing a product's toxicity. **The** composition of the present invention will have a total PAH content of no more than 20 TEQ B[a]P, wherein this total TEQ value corresponds to the sum of TEQs for four specific PAHs, namely benzo[a]pyrene, benzo[a]anthracene, benzo[b]fluoranthene and chrysene (this is referred to as the 4-PAH 35 method). In particular, when the antimicrobial composition of the present invention is produced from coconut oil, the total PAH content will be no higher than 20 TEQ B[a]P. If it is produced from palm kernel oil, it will preferably be no higher than 10 TEQ B[a]P. Preferably, the composition of the present invention with have a total PAH content of no more than 10, more preferably no more than 4, more preferably no more than 1 TEQ B[a]P. In addition, the composition of the present invention will preferably have a B[a]P content of no more than 2, 5 more preferably no more than 1 TEQ.

The total dioxin content of the compositions of the present invention will preferably be no more than 1.5, more preferably no more than 1 TEQ, more preferably no more than 0.75 TEQ - corresponding to the sum of TEQs for polychlorinated biphenyls (PCB), polychlorinated dibenzo-p-dioxins (PCDD) and polychlorinated dibenzofurans (PCDF). Furthermore, the composition of the invention will preferably have a PCB level no higher than 0.75, more preferably no higher than 0.5 TEQ, and a PCDD+PCDF level preferably no higher than 0.75, more preferably no higher than 0.5 TEQ.

The compositions of the present invention are suitable for use in food, feed and beverage compositions. In particular, they are suitable for use as antimicrobial agents for animal feed compositions and, in fact, animal feed compositions comprising such antimicrobial compositions are also part of the present invention.

The term "animal feed composition" as used herein, includes all solid or semi-solid feed compositions as well as liquid feed compositions and pre-mixes. It covers both agricultural feeds and pet foods. The animal feed composition will be admixed with the antimicrobial composition of the present invention to form an animal feed composition which, when administered, will provide an effective amount of the antimicrobial composition to the animal.

According to one particular embodiment, the present invention relates to feed compositions comprising the antimicrobial composition as defined in claim 1 in an amount of 0.05 to 10% by weight, preferably in an amount of 0.2 to 5% by weight, even more preferably in an amount of 0.3 to 2% by weight, for example in an amount of 0.5 to 1% by weight, based on the total weight of the final feed.

In addition to the antimicrobial composition, the animal feeds of the present invention may further comprise one or more other active ingredients. These may include any material which can be added to the feed to enhance the animal's health, performance, and/or well-being.

Examples of such ingredients are referred to in "2006 Feed Additive Compendium" and "Handbook of Feed Additives 2006".

Feed compositions of the present invention may be used, for instance, for equine animals (such as horses), ovine animals (such as lamb and sheep) and bovine animals (such as cattle), but will be particularly suitable for calves (e.g. veal calves), porcine animals (such as pigs and piglets), rabbits, poultry (such as chickens, turkeys, ducks, pheasant and quail), domestic animals (such as cats and dogs), and aquatic animals (such as fish and shrimp).

The antimicrobial compositions of the present invention, and animal feed compositions comprising them, may be used to enhance feed efficiency and/or enhance growth and/or reduce mortality in animals. Thus, the present invention further provides a method of enhancing feed efficiency and/or enhancing growth and/or reducing mortality in an animal comprising providing to said animal for an effective time an effective amount of the antimicrobial composition of the present invention.

Feed efficiency is a term generally known in the art and refers to a ratio of weight of food ingested/weight gain of an animal (F:G). Enhancement of feed efficiency is an overall decrease in this F:G ratio over that which would otherwise occur without implementation of the methods and/or administration of the compositions of the present invention.

Growth and enhancing growth are terms generally known in the art and refer to increases in either, or both, weight and size (e.g., height, width, diameter, circumference, etc.) over that which would otherwise occur without implementation of the methods and/or administration of the compositions of the present invention. Growth can refer to an increase in the mass (e.g., weight or size) of the entire animal or of a particular tissue (e.g., muscle tissue in general or a specific muscle). Alternatively, growth can indicate a relative increase in the mass of one tissue in relation to another, in particular, an increase in muscle tissue relative to other tissues (e.g., adipose tissue).

Reducing mortality refers to increasing the survivability or decreasing the death rate in animals after birth or hatch as compared with that which would otherwise occur in the absence of implementation of the methods and/or administration of the compositions of the present invention.

Effective amount refers to the amounts of administration of the antimicrobial composition, to provide enhanced growth, enhanced feed efficiency, and/or reduced mortality. Further, such amounts and rates should result in no or few adverse events in the treated animal. As those
familiar with the art will understand, the amounts and rates will vary depending upon a number of factors. These factors include, for example, the type of animal being treated, its weight and general physical condition, and the dosing regimen. Ranges for the rate of administration of the antimicrobial composition are from about 1 to about 3000, desirably 10 to 1000, and more desirably from about 10 to about 500 mg/kg of weight of the animal. These amounts are to be administered normally every day for at least 7 days, at least 2 weeks, at least 30 days, over 60 days, over 100 days, or for all or a substantial portion of the life of the animal.

The term "food composition" as used herein, will include all food products suitable for consumption by humans, including infants, children and the elderly, but will also cover compositions used as nutritional supplements whether provided in liquid or solid form. The antimicrobial compositions of the present invention have indeed been found to have a probioticlike effect, enabling the growth of positive bacteria in the gut by reducing or inhibiting the growth of negative bacteria. Thus food compositions of the present invention may contribute to improved gut function.

## EXAMPLES

### Example 1 - Preparation of a Split Coconut Oil

A crude coconut oil from the Philippines, with 3% free fatty acids (FFA), was physically refined. The physical refining process consisted of an acid degumming step (citric acid), a bleaching step (with 1.5% acid activated bleaching earth and 2.5 kg/MT Norit SA Ultra activated carbon), and, finally, a deodorization step (240C, 1% steam, 3 mbar). All conditions and filter aid used are according to industrial standards. The refined oil had a FFA level of < 0.1% and a Lovibond red of <2.

The activated carbon treated oil was then hydrolysed using the Colgate-Emery process to obtain 98% free fatty acid purity, measured according to AOCS method Ca 5a-40 (lauric acid). The conditions of the Colgate-Emery process were according to industrial standards.

### Example 2 - Removal of PAHs using activated carbon

Crude coconut oil containing 105 ppb of 4-PAH was treated with active carbon and physically refined under similar conditions as those described in Example 1. After the bleaching and
activated carbon treatment step, the oil contained 3 ppb PAH and after the final deodorization step the PAH level was 0.3 ppb.

### Example 3 - Effects of Oil Source on Production Performance and Digestibility

*Experimental Design*

A trial to evaluate the effect of different oil sources (soya oil and CNO-split obtained according to Example 1) was performed over a period of just over a month, split into two phases: Phase 1 (0- 14 days), the starter phase, and Phase 2 (14-35 days), the grower phase. At 14 days, all birds were switched to a grower diet until 34 days of age. Feed and water were provided ad libitum.

*Birds and Housing*

A total of 420 male Ross 308 day-old male chicks, derived from 57 week old broiler breeders, were purchased from a commercial hatchery. On arrival, the birds were randomly assigned to cages, with 17 birds per cage. After placement of the chicks, the total weight of all birds per cage was recorded to determine start weight of the chicks. The cages were evenly divided between two rooms (A and B). The cages in room A had average starting weights of 47.9 (+/- 1.1) g/chick. The cages in room B has average starting weights of 44.5 (+/-1.3) g/chick.

Throughout the trial, birds were housed in individual broiler grower cages (100 x 110 cm) on litter (wood shavings). Each cage was equipped with two drinking cups with adjustable height. For the first 14 days, the feeder was inside the cage, and thereafter the feed was supplied via a feeder through the front of the cage.

Day length was set for 23 hours a day during the first 3 days, 20 hours a day from day 4 until day 7, and 18 hours a day during the remainder of the trial. Temperature, humidity and ventilation were computer controlled. Temperature was gradually decreased 2.5°C per week, from 35°C on the day of arrival to a final temperature of 20.5°C at the end of the experiment (day 35). Relative humidity was set at 50%. The birds were spray-vaccinated against Newcastle Disease (Poulvac NDW-vaccine, Intervet, Boxmeer, NL) at 10 days of age.

*Experimental Diets*

In advance of diet formulation, batches of wheat, corn and soybean meal (SBM) were reserved and wet chemically analysed for crude protein. Additionally, SBM was analysed for dry matter and K. Near Infrared Spectroscopy (NIRS) was used to predict crude ash, crude fat, crude fiber and moisture. SBM was also analysed for amino acid content.

Formulation of diets was based on the analysed nutrient content of the reserved raw materials. A basal diet was formulated, with soya oil as the only fat source. The other fat source (CNO-Split, prepared in accordance with Example 1) was added on a weight to weight basis, fully replacing the soya oil. The composition of the experimental diets is given in the following tables.

Table 1 - Ingredients

| Ingredients, % | Starter diet | Grower diet |
| --- | --- | --- |
| Corn BG1205 | 41.9 | 45.0 |
| Wheat BG1205 | 15.0 | 15.0 |
| Soybean meal >48% BG1205 | 33.7 | 30.6 |
| Fats/oils, Soya oil | 4.51 | 4.64 |
| Limestone | 1.7 | 1.4 |
| Monocalciumphosphate | 1.32 | 1.11 |
| Broiler premix 1% | 1.00 | 1.00 |
| Sodiumbicarbonate | 0.30 | 0.23 |
| Salt | 0.178 | 0.174 |
| L-Lysine HCl | 0.162 | 0.171 |
| DL-Methionine | 0.197 | 0.179 |
| L-Threonine | 0.010 | 0.011 |
| Total | 100.0 | 100.0 |

Table 2 - Nutrients

| Nutrients, % | Starter diet | Grower diet |
| --- | --- | --- |
| Crude protein | 21.5 | 20.4 |
| OIL (EE) | 7.1 | 7.3 |
| Crude fibre | 2.5 | 2.4 |
| Ash | 6.2 | 5.5 |

(continued)

| Nutrients, % | Starter diet | Grower diet |
|---|---|---|
| DM | 88.7 | 88.6 |
| Calcium | 0.97 | 0.80 |
| Phosphor, total | 0.68 | 0.62 |
| Na | 0.16 | 0.14 |
| K | 0.88 | 0.83 |
| Cl | 0.18 | 0.18 |
| Dig.P broilers | 0.40 | 0.35 |
| aP broilers | 0.43 | 0.38 |
| dEB, meq | 243 | 223 |
| AME poultry, kcal | 3053 | 3120 |
| AME poultry (FS-R), kcal | 3068 | 3134 |
| AME broiler, kcal | 2850 | 2925 |
| LYS | 1.22 | 1.16 |
| MET | 0.52 | 0.49 |

Table 3 - Analysis

| | Starter diets | | Grower diets | |
|---|---|---|---|---|
| | Control | CNO | Control | CNO |
| *Calculated nutrients, %* | | | | |
| Crude protein | 21.5 | 21.5 | 20.4 | 20.4 |
| Crude fat | 7.1 | 7.1 | 7.3 | 7.3 |
| Crude fibre | 2.5 | 2.5 | 2.4 | 2.4 |
| Dry matter | 88.7 | 88.7 | 88.6 | 88.6 |
| Ca | 0.97 | 0.97 | 0.80 | 0.80 |
| P | 0.68 | 0.68 | 0.62 | 0.62 |
| *Analysed nutrients* | | | | |
| Crude protein | 22.3 | 22.5 | 21.1 | 21.0 |
| Crude protein (NIRS) | 22.5 | 22.9 | 21.2 | 21.4 |
| Crude fat (Soxhlet extraction) | 7.0 | 6.2 | 7.2 | 6.9 |
| Crude fat (NIRS) | 7.0 | 6.8 | 7.2 | 7.0 |
| Crude fibre (NIRS) | 2.3 | 1.9 | 2.4 | 1.9 |
| Moisture (NIRS) | 10.6 | 10.7 | 11.2 | 10.7 |
| Dry matter | 89.4 | 89.3 | 88.8 | 89.3 |
| Ca | 0.98 | 0.99 | 0.81 | 0.84 |
| P | 0.65 | 0.65 | 0.59 | 0.60 |
| *% of expected* | | | | |
| Crude protein | 104 | 105 | 103 | 103 |

The diets were produced by Research Diet Services, the Netherlands. First a batch of basal diet

| Crude fat | 99 | 88 | 98 | 94 |
| Crude fibre | 91 | 75 | 99 | 78 |
| Dry matter | 101 | 101 | 100 | 101 |
| Ca | 101 | 102 | 102 | 105 |
| P | 96 | 96 | 95 | 96 |

without soya oil was produced. Then, to each batch the fat source (soya oil or CNO-split) was added. Starter diets were pelleted at 2.5 mm and grower diets at 3 mm with steam addition (app. 5 80°C).

*Data Collection*

Bird weights were recorded per cage at the start of the experiment (day 0) and individually at 7, 10 14, 21, 28 and 35 days of age. In addition, feed consumption for each cage was recorded on the same day the birds were weighed. Based on calculated body weight gain and feed consumption, feed to gain ratio (F:G) was calculated as kg of feed consumed / kg of weight gain. Total feed consumption per cage was corrected for mortality, culling and outliers (table 5). Finally, the European Poultry Index (EPI - De Herdt et all., 1999) was calculated using the following formula: 15

EPI = (final body weight (g) x (100% - mortality%)) / ((10 x period in days) x overall FCR), where FCR = Feed Conversion Ratio.

The EPI excluding mortality was calculated using the following formula:

EPI = (final body weight (g) x 100) / ((10 x period in days) x overall FCR)

The results are set out in the following table.

Table 4 - Body Weight and Feed Consumption

| | Soya oil | CNO-Split |
|---|---|---|
| Body Weight (BW) at 0 days, in g | 46 | 46 |
| BW 14d, g | 553 | 543 |
| BW 34d, g | 2353 | 2441 |
| Average Daily Gain (ADG) from 0-7 days, in g | 20.5 | 20.7 |
| ADG 0-14d, g | 36.2 | 35.5 |
| ADG 0-34d, g | 67.8 | 70. 5 |
| ADG 14-34d, g | 90.0 | 94.9 |
| Average Daily Feed Intake (ADFI) from 0-7 days, in g | 19.7 | 19.5 |
| ADFI 0-14d, g | 39.7 | 39.1 |
| ADFI 0-34d, g | 97.5 | 98.7 |
| ADFI 14-34d, g | 138 | 140 |
| Feed to Gain ratio (F:G) from 0-7 days, in g | 0.958 | 0.939 |
| F:G 0-14d, g | 1.097 | 1.102 |
| F:G 0-34d, g | 1.438 | 1.401 |

(continued)

| | Soya oil | CNO-Split |
|---|---|---|
| F:G 14-34d, g | 1.534 | 1.479 |
| F:G corrected for final BW | 1.432 | 1.369 |
| European Performance Index (EPI) | 458 | 493 |
| EPI, excluding mortality | 481 | 513 |

Table 5 - Mortality, culling and Outliers

| Period | Mortality | Culling | Outliers* | Total** |
|---|---|---|---|---|
| d 0-14 | 2.0 | 0.2 | 2.2 | 4.4 |
| d 14-34 | 0.2 | 3.2 | 2.0 | 5.4 |
| d 0-34 | 2.2 | 3.4 | 4.2 | 9.8 |
| * birds with lower weights than the [(average cage weight) - (2.5 x cage SD)] and female birds (d 34); ** calculated as a percentage of birds on d 0. | | | | |

At day 35 of the experiment, all birds of each cage were successively weighted and killed by $CO_2/O_2$. Subsequently, the intestinal tract was removed and the content of the 2nd half of the ileum (middle of ileum to the ileal-cecal-colon junction) was collected by gently squeezing using fingers. Samples were held on ice, frozen (-18°C) and, afterwards, freeze-dried, ground (0.5 mm screen) and stored for analysis.

All samples were analysed for crude fat (AOCS Am 5-04), gross energy (bomb calorimetry) and acid insoluble ash (Schothorst Feed Research, the Netherlands) to determine digestibility.

*Results*

Nutritional composition of the diets was in line with the expected values, except crude fat levels 5 of the CNO-split starter diet which were lower than expected. This may be due to the MCFA content of the CNO-split for which a regular crude fat analysis is not suitable as heating of the sample during preparation may result in evaporation of the MCFA. During pelleting, heat is also used and this may also cause some evaporation.

Observed health status of the birds was good throughout the experiment. Mortality, including culling, reached 5.6%. No statistical differences in mortality were found between the different diets (i.e. soya oil vs. CNO-split).

Technical performance (mean body weight at d 34: 2,389 g) was in line with other trials carried 15 out at the same facilities (mean body weight for 2011: 2,444 g).

During the starter period (0-14d), there was no effect of fat source on average daily gain (ADG) or average daily feed intake (ADFI). However, F:G was affected by the fat source in the diet. From 0 to 7 days, CNO-split numerically improved F:G by 2.0% compared to soya oil. This may 20 be related to the poor fat digestion of young birds, with MCFA being a more easily digested energy source.

In the grower period (14-34d) ADG was significantly higher for birds fed CNO-split (+5.5%). Additionally, CNO-split significantly improved F:G (by 3.6%) compared to soya oil. During the 25 entire period (0-34d), similar trends as for the grower period were observed: CNO-split significantly improved ADG (by 3.9%) and overall period F:G (by 2.6%).

The only difference in excrecia quality was a tendency for the CNO-split to lower free water. This is thought to be related to an improved intestinal health.

**Claims**

1. An antimicrobial composition obtainable by a method comprising the step of splitting a refined lauric oil **characterized in that** the refined oil is an oil that has been brought into contact with activated carbon saicl composition comprising 90% or more, by weight, free fatty acids,
FFAs, and **characterized in that** it comprises, by weight:

   • 0-5% C6 fatty acids;

• 1 -15% C8 fatty acids;
• 1-15% C10 fatty acids; and
• 35-70% C12 fatty acids; and having
• a total polycyclic aromatic hydrocarbons (PAH) level of 20 toxicity equivalence of sum of benzo[a]pyrene, benzo[a]anthracene, benzo[b]fluorthene and chrysene (TEQ B[a]P),

or less.

2. The antimicrobial composition according to claim 1, **characterized in that** it has

• a total dioxin content no greater than 1.5 toxicity equivalence of 2,3,7,8-tetrachlorodibenzodioxin (TEQ).

3. The antimicrobial composition according to claim 1 or 2 **characterized in that** it comprises 95% or more, preferably 97% or more, free fatty acids, FFAs, by weight.

4. The antimicrobial composition according to anyone of claims 1 to 3 **characterized in** the refined oil is an oil that has been degummed, bleached and deodorized.

5. The antimicrobial composition according to anyone of claimsl to 4 **characterized in that** the splitting step is selected from chemical hydrolysis and enzymatic splitting.

6. The antimicrobial composition according to any one of claims 1 to 5 for use in preventing the growth of microbial agents and/or for improving gastro-intestinal health in animals.

7. The antimicrobial composition according to anyone of claims 1 to 5, for use in increasing feed efficiency and/or enhancing growth and/or reducing mortality in animals.

8. A feed comprising the composition according to any one of claims 1 to 7, preferably in an amount of 0.05-10% by weight.

9. The feed according to claim 8, suitable for use with calves, porcine animals, rabbits, poultry, domestic animals, and aquatic animals.


**Patentansprüche**

1. Antimikrobielle Zusammensetzung, erhältlich durch ein Verfahren, umfassend den Schritt des Aufspaltens eines raffinierten Laurinöls, **dadurch gekennzeichnet, dass** das raffinierte Öl ein Öl ist, das mit Aktivkohle in Kontakt gebracht worden ist, wobei die Zusammensetzung 90 Gew.-% oder mehr freie Fettsäuren, FFAs, umfasst und **dadurch gekennzeichnet ist, dass** sie umfasst, auf Gewichtsbasis :

• 0 - 5 Gew.-% C6 Fettsäuren;
• 1 - 15 Gew.-% C8 Fettsäuren;
• 1 - 15 Gew.-% C10 Fettsäuren; und
• 35 - 70 Gew.-% C12 Fettsäuren; und aufweisend
• einen Gesamtpegel polyzyklischer aromatischer Kohlenwasserstoffe (PAH) von 20 Toxizitätsäquivalenten aus der Summe aus Benzo[a]pyren, Benzo[a]anthracen, Benzo[b]fluorthen und Chrysen (TEQ B[a]P) oder weniger.

2. Antimikrobielle Zusammensetzung nach Anspruch 1, **dadurch gekennzeichnet, dass** diese

• einen Gesamtdioxingehalt von nicht mehr als 1,5 Toxizitätsäquivalenten aus 2,3,7,8-Tetrachlordibenzodioxin (TEQ) aufweist.

3. Antimikrobielle Zusammensetzung nach Anspruch 1 oder 2, **dadurch gekennzeichnet, dass** diese 95 Gew.-% oder mehr, bevorzugt 97 Gew.-% oder mehr, freie Fettsäuren, FFAs, umfasst.

4. Antimikrobielle Zusammensetzung nach einem der Ansprüche 1 bis 3, **dadurch gekennzeichnet, dass** das raffinierte Öl ein Öl ist, das entschleimt, gebleicht und deodorisiert worden ist.

5. Antimikrobielle Zusammensetzung nach einem der Ansprüche 1 bis 4, **dadurch gekennzeichnet, dass** der Aufspaltungsschritt ausgewählt ist aus chemischer Hydrolyse und enzymatischem Aufspalten.

6. Antimikrobielle Zusammensetzung nach einem der Ansprüche 1 bis 5, zur Anwendung bei dem Verhindern des Wachstums von mikrobakteriellen Mitteln und/oder zum Verbessern der Gastro-intestinalen Gesundheit bei Tieren.

7. Antimikrobielle Zusammensetzung nach einem der Ansprüche 1 bis 5, zur Anwendung bei dem Erhöhen der Füttereffizienz und/oder dem Unterstützen des Wachstums und/oder Verringern der Sterblichkeit bei Tieren.

8. Futtermittel, aufweisend die Zusammensetzung nach einem der Ansprüche 1 bis 7, vorzugsweise in einer Menge von 0,05 - 10 Gew.-%.

9. Futtermittel nach Anspruch 8, geeignet zur Anwendung bei Kälbern, Schweinen, Hasen, Geflügel, Haustieren und Wassertieren.

**Revendications**

1. Composition antimicrobienne pouvant être obtenue par un procédé comprenant l'étape de fractionnement d'une huile laurique raffinée **caractérisée en ce que** l'huile raffinée est une huile qui a été mise en contact avec du charbon actif, ladite composition comprenant 90% ou plus, en poids, d'acides gras libres, les FFA, et **caractérisée en ce qu'**elle comprend, en poids :

   - 0 à 5 % d'acides gras en C6 ;
   - 1 à 15 % d'acides gras en C8 ;
   - 1 à 15 % d'acides gras en C10 ; et
   - 35 à 70 % d'acides gras en C12 ; et présentant
   - un taux total d'hydrocarbures aromatiques polycycliques (PAH) de 20 en termes d'équivalence toxique pour la somme du benzo[a]pyrène, du benzo[a]anthracène, du benzo[b]fluorthène et du chrysène (TEQ B[a]P), ou moins.

2. Composition antimicrobienne selon la revendication 1, **caractérisée en ce qu'**elle présente

   - une teneur totale en dioxine qui n'est pas supérieure à 1,5 en termes d'équivalence toxique pour la 2,3,7,8-tétrachlorodibenzodioxine (TEQ).

3. Composition antimicrobienne selon la revendication 1 ou 2, **caractérisée en ce qu'**elle comprend 95 % ou plus, de préférence 97 % ou plus, d'acides gras libres, les FFA, en poids.

4. Composition antimicrobienne selon l'une quelconque des revendications 1 à 3 **caractérisée en ce que** l'huile raffinée est une huile qui a été dégommée, décolorée et désodorisée.

5. Composition antimicrobienne selon l'une quelconque des revendications 1 à 4 **caractérisée en ce que** l'étape de fractionnement est sélectionnée parmi l'hydrolyse chimique et le fractionnement enzymatique.

6. Composition antimicrobienne selon l'une quelconque des revendications 1 à 5 pour son utilisation dans la prévention de la croissance d'agents microbiens et/ou pour améliorer la santé gastro-intestinale chez les animaux.

7. Composition antimicrobienne selon l'une quelconque des revendications 1 à 5, pour son utilisation dans l'augmentation du rendement alimentaire et/ou pour améliorer la croissance et/ou pour réduire la mortalité chez les animaux.

8. Produit alimentaire comprenant la composition selon l'une quelconque des revendications 1 à 7, de préférence en une quantité de 0,05 à 10 % en poids.

9. Produit alimentaire selon la revendication 8, approprié pour une utilisation avec les veaux, les animaux porcins, les lapins, les volailles, les animaux domestiques, et les animaux aquatiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5142071 A
- WO 2011012440 A2
- US 2012041065 A1
- US 4223040 A

**Non-patent literature cited in the description**

- Developments in Edible Oil Refining for the Production of High Quality Food Oils. *101st AOCS ANNUAL MEETING*
- *Antimicrobial agents and chemotherapy,* vol. 2 (1), 23-28
- *Polish Journal of Microbiology,* vol. 58 (1), 43-47